(19) Europäisches Patentamt
European Patent Office
Office européen des brevets

(11) **EP 2 010 060 B1**

(12) **EUROPEAN PATENT SPECIFICATION**

(45) Date of publication and mention
of the grant of the patent:
**09.02.2011 Bulletin 2011/06**

(51) Int Cl.:
***A61B 8/00*** (2006.01)

(21) Application number: **07746130.9**

(22) Date of filing: **23.04.2007**

(86) International application number:
**PCT/KR2007/001968**

(87) International publication number:
**WO 2007/123352 (01.11.2007 Gazette 2007/44)**

(54) **ULTRASONIC DIAGNOSIS APPARATUS FOR AN URINARY BLADDER AND THE METHOD THEREOF**

ULTRASCHALLDIAGNOSEVORRICHTUNG FÜR EINE BLASE UND VERFAHREN DAVON

APPAREIL DIAGNOSTIQUE ULTRASONIQUE DESTINÉ À LA VESSIE ET PROCÉDÉ CORRESPONDANT

(84) Designated Contracting States:
**DE FR GB IT NL SE**

(30) Priority: **25.04.2006 KR 20060037132**

(43) Date of publication of application:
**07.01.2009 Bulletin 2009/02**

(73) Proprietor: **Mcube Technology Co., Ltd.**
**Chungnang-gu, Seoul**
**131-221 (KR)**

(72) Inventors:
• **KIM, Jung-Hoe**
**Seoul 131-140 (KR)**

• **KIM, Seung-Tai**
**Gwacheon-si**
**Gyeonggi-do 427-806 (KR)**

(74) Representative: **Viering, Jentschura & Partner**
**Postfach 22 14 43**
**80504 München (DE)**

(56) References cited:
**JP-A- 05 237 112     JP-A- 2003 190 168**
**US-A- 4 926 871     US-A- 5 964 710**
**US-A- 6 110 111     US-A1- 2004 267 123**
**US-B1- 6 213 949**

**Description**

[Technical Field]

The present invention relates generally to a portable ultrasonic diagnosis apparatus for the urinary bladder and an ultrasonic diagnosis method using the apparatus and, more particularly, to a portable and small-sized ultrasonic diagnosis apparatus, which has a preliminary scan mode and a scan mode, thus not only quickly and accurately detecting the location of the urinary bladder but also automatically measuring the amount of urine in the urinary bladder, and an ultrasonic diagnosis method, which can measure the amount of urine in the urinary bladder using the apparatus.

[Background Art]

Generally, an ultrasonic system is a system that emits ultrasonic signals to an object to be examined using the piezoelectric effect of a transducer, receives the ultrasonic signals reflected from the discontinuous planes of the object, converts the received ultrasonic signals into electrical signals, and outputs the electrical signals to a predetermined display device, thus enabling examination of the internal state of the object. Such an ultrasonic system is widely used for medical diagnosis equipment, non-destructive testing equipment and underwater detection equipment.

However, most conventional ultrasonic diagnosis apparatuses are inconvenient in that they cannot be easily carried due to their large size and heavy weight. To solve the inconvenience, various portable ultrasonic diagnosis apparatuses have been proposed. Korean Utility Model Registration No. 20-137995 discloses a "Portable Ultrasonic Diagnosis Apparatus."

Meanwhile, when examining bladder abnormalities or urinary difficulty, measuring the amount of urine is an essential procedure. Furthermore, prior to urination using a catheter, the amount of urine in the urinary bladder should be measured to account for urine that may be retained after the operation. In addition, in urination training, the amount of urine in the urinary bladder should be measured as a guideline.

Various types of ultrasonic scanning equipment may be used to measure the amount of urine in the urinary bladder, as described above. In this case, two methods are used. A first method calculates the amount of urine from respective ultrasonic images for a perpendicular plane and a horizontal plane, which are obtained using typical ultrasonic scanning equipment. However, although many algorithms has been proposed and used for the method, the first method is problematic in that it not only exhibits a considerable error rate but also exhibits different results for different users. A second method uses dedicated ultrasonic equipment for measuring the amount of urine. U.S. Pat. No. 4,926,871 discloses dedicated ultrasonic equipment. However, the dedicated ultrasonic equipment based on the second method has a disadvantage in that it also calculates the amount of urine chiefly using two ultrasonic images, which are related to the perpendicular and horizontal planes of the urinary bladder, respectively, and in that a user must find the area indicating the greatest size and select it in order to calculate the amount of urine.

Accordingly, the present applicant proposes a method of accurately calculating the amount of urine in the urinary bladder while minimizing user interference.

Document US 2004/267123 A1 discloses to operate an ultrasonic diagnosis apparatus using two different operational modes the first mode scanning only a single plane of an urinary bladder, and the second mode successfully scanning a plurality of plane of the urinary bladder so as to carry out a pre-adjustment of the apparatus using the first operational mode at high speed and after this a detailed scanning of the urinary bladder using the second operational model at low speed.

[Disclosure]

[Technical Problem]

In order to solve the above problems, an object of the present invention is to provide an ultrasonic diagnosis apparatus for the urinary bladder, which can not only quickly and accurately detect the location of the urinary bladder but also measure the amount of urine in the urinary bladder.

Another object of the present invention is to provide an ultrasonic diagnosis apparatus for the urinary bladder, which has a size and weight suitable for portable applications.

A further object of the present invention is to provide an ultrasonic diagnosis method in which the ultrasonic diagnosis apparatus thereof can accurately measure the amount of urine in the urinary bladder using received ultrasonic signals.

[Technical Solution]

In order to accomplish the above objects, the present invention provides an ultrasonic diagnosis apparatus for an urinary bladder according to claim 1. The ultrasonic diagnosis apparatus measures the amount of urine in the urinary bladder and includes:

a transducer for emitting ultrasonic signals and receiving ultrasonic signals reflected from an object;
a transducer support configured such that the transducer is fixedly installed therein;
an analog signal processing unit for converting the ultrasonic signals, which are transmitted from the transducer, into digital signals;
a display unit for outputting specific image signals; a central control unit for performing image processing on the digital ultrasonic signals transmitted from the analog signal processing unit, outputting the results of the processing to the display unit, and controlling the overall operation of the apparatus;
a first stepping motor for rotating the transducer in a first direction;
a second stepping motor for rotating the transducer in a second direction;
a drive control unit for controlling the operation of the first and second stepping motors in response to drive control signals provided from the central control unit; and
a switch unit for selecting operation modes;
wherein, when a first operational mode is selected by the switch unit, the central control unit receives pieces of ultrasonic information of n scan lines for a single plane at a current location from the transducer, acquires an image from the pieces of received ultrasonic information, and outputs the acquired image to the display unit, and
when a second operational mode is selected by the switch unit, the central control unit receives pieces of ultrasonic information of n scan lines for each of m planes from the transducer, and calculates the amount of urine in the urinary bladder using the pieces of received ultrasonic information.

In the ultrasonic diagnosis apparatus, when the first operational mode is selected, it is preferred that the central control unit transmits a drive control signal for rotating the second stepping motor at a current location to the drive control unit,

the drive control unit sequentially rotates the second stepping motor in response to the drive control signal received from the central control unit, and
the central control unit receives the pieces of ultrasonic information of n scan lines, which are transmitted from the transducer, according to the second stepping motor, extracts a two-dimensional bladder image for a corresponding plane from the pieces of received ultrasonic information, and outputs the extracted two-dimensional bladder image to the display unit.

In the ultrasonic diagnosis apparatus, when the second operational mode is selected, the central control unit sequentially rotates the transducer in the first direction by rotating the first stepping motor, and transmits a drive control signal, which is used to rotate the second stepping motor in the second direction by a predetermined angle n times, to the drive control unit whenever the first stepping motor rotates,

the drive control unit rotates the first and second stepping motors in response to the drive control signals transmitted from the central control unit, and
the central control unit calculates the amount of urine in the urinary bladder using the pieces of ultrasonic information of n scan lines for each of m planes, which are sequentially received from the transducer according to the rotation of the first and second stepping motors.

In addition, the present invention provides an ultrasonic diagnosis method, according to claim 4. The ultrasonic diagnosis method measures amount of urine in the urinary bladder using an ultrasonic diagnosis apparatus and includes the steps of:

(a) determining an operational mode input from an outside;
(b) if it is determined that the operational mode input from the outside is a preliminary scan mode, receiving pieces of ultrasonic information of n scan lines for a single plane at a current location from a transducer, extracting a bladder image for a corresponding plane from the pieces of received ultrasonic information, and outputting the extracted image to a display unit; and
(c) if it is determined that the operational mode input from the outside is a scan mode, sequentially receiving pieces of ultrasonic information of n scan lines for each of m planes from the transducer, and measuring the amount of urine in the urinary bladder using the pieces of received ultrasonic information.

In the ultrasonic diagnosis method, the step (c) include the steps of:

(c1) detecting the locations of front and rear walls from the pieces of ultrasonic information of all of the scan lines;
(c2) obtaining difference values between the detected locations of the front and rear walls for the respective scan lines;
(c3) obtaining areas for bladder images of the respective planes using the difference values for the scan lines of each plane;
(c4) obtaining correction coefficients for the respective planes;
(c5) calculating radii of respective circles having areas identical to areas for the bladder images of the respective planes, and calculating corrected radii by applying the correction coefficients for the respective planes to the radii for the respective planes;
(c6) obtaining an average radius of the corrected radii for the respective planes; and

(g) obtaining the volume of a sphere using the average radius. In this case, the finally obtained volume of the sphere is the volume of urine in the urinary bladder.

[Advantageous Effects]

According to the present invention, two stepping motors having one transducer and two rotational axes are provided, so that an ultrasonic diagnosis apparatus that not only has small size and weight but also can provide ultrasonic information about a three-dimensional image.
Furthermore, the two stepping motors of the ultrasonic diagnosis apparatus according to the present invention collect the ultrasonic information while rotating automatically, so that all of the ultrasonic information included in a cone-shaped region from the location at which the ultrasonic diagnosis apparatus is disposed can be collected. As a result, conventional apparatuses measure the amount of urine in the urinary bladder using only ultrasonic information about two planes, and thus data is incorrect, whereas the apparatus according to the present invention measures the amount of urine using ultrasonic information about a plurality of planes that are uniformly spaced throughout 360°, so that it can very accurately measure the amount of urine.
In particular, the apparatus according to the present invention uses correction coefficients that numerically indicate the extent to which the first detected location is displaced from the center of the urinary bladder, so that accurate measurement can be always performed even if the detected location is displaced from the center of the urinary bladder.
Furthermore, the ultrasonic diagnosis apparatus according to the present invention operates in the preliminary scan mode, and thus the central location of the urinary bladder that a user desires to examine can be quickly and accurately detected. As a result, the amount of urine in the urinary bladder can also be quickly and accurately measured.

[Description of Drawings]

FIG. 1 is a block diagram schematically showing the internal construction of an ultrasonic diagnosis apparatus according to a preferred embodiment of the present invention;
FIG. 2 is a perspective view showing the ultrasonic diagnosis apparatus of FIG. 1;
FIG. 3 is a conceptual diagram illustrating a process of acquiring a two-dimensional image using the ultrasonic diagnosis apparatus of FIG. 2; and

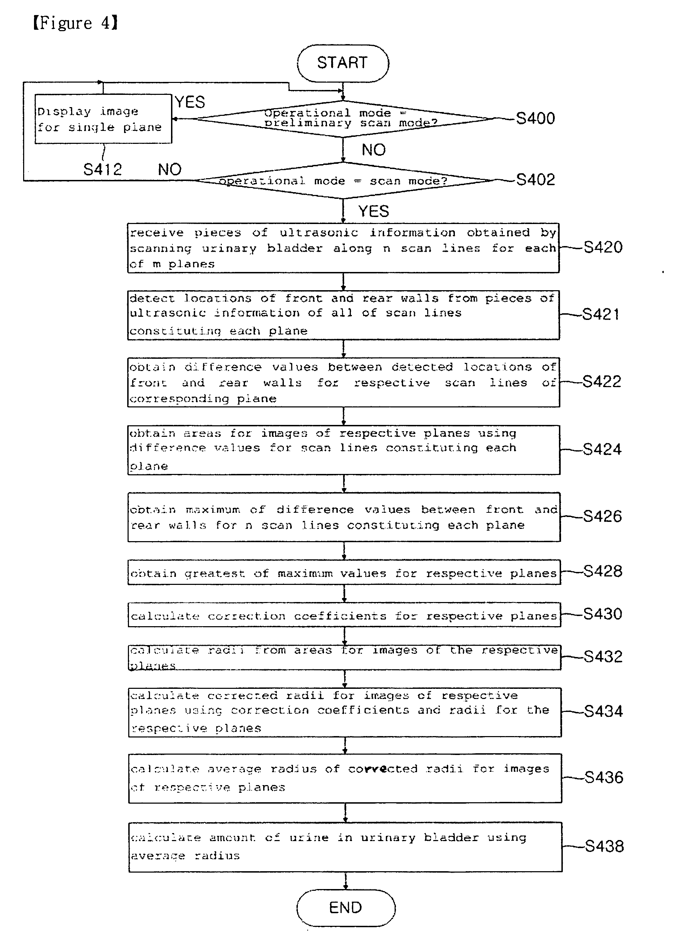

FIG. 4 is a flowchart sequentially illustrating a process of obtaining the volume of urine in the urinary bladder using the ultrasonic diagnosis apparatus according to a preferred embodiment of the present invention.

[Best Mode]

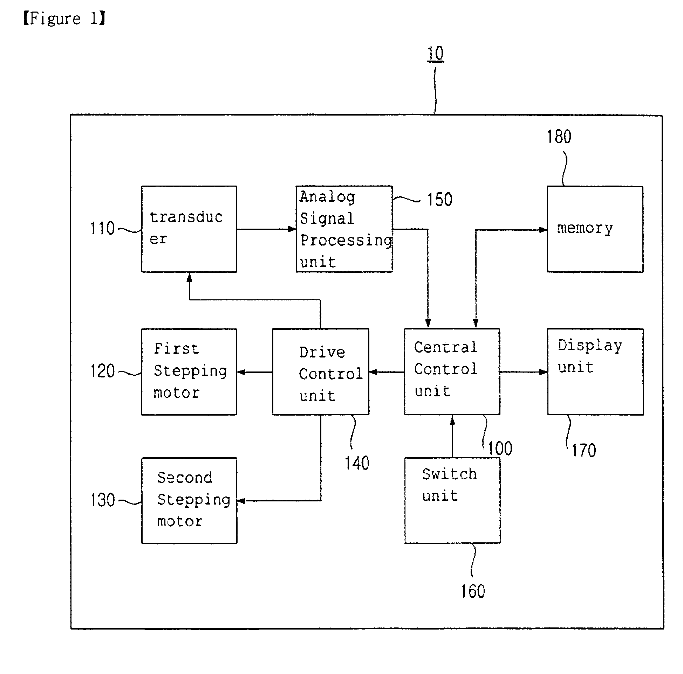

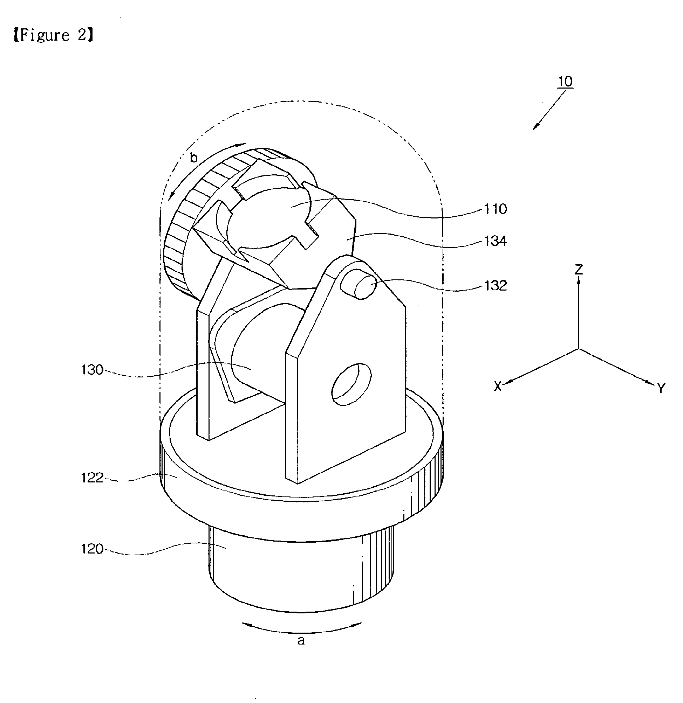

The construction and operation of an ultrasonic diagnosis apparatus for the urinary bladder according to a preferred embodiment of the present invention are described in detail with reference to the accompanying drawings below. FIG. 1 is a block diagram schematically showing the internal construction of an ultrasonic diagnosis apparatus according to the preferred embodiment of the present invention, and FIG. 2 is a perspective view showing the ultrasonic diagnosis apparatus of FIG. 1.
Referring to FIG. 1, the ultrasonic diagnosis apparatus 10 according to the preferred embodiment of the present invention includes a central control unit 100 for controlling the overall operation of the apparatus, a transducer 110, a first stepping motor 120, a second stepping motor 130, a drive control unit 140, an analog signal processing unit 150, a switch unit 160, memory 180, and a display unit 170. The respective components of the above-described ultrasonic diagnosis apparatus 10 are described in detail below.

The transducer 110 is a device that emits ultrasonic signals and receives ultrasonic signals reflected from the internal organs of a human body, and transmits the received analog signals to the analog signal processing unit 150. The transducer 110 of the ultrasonic diagnosis apparatus for the urinary bladder according to the present invention receives ultrasonic signals reflected from urine in the urinary bladder.

The analog signal processing unit 150 converts the analog signals, which are transmitted from the transducer 110, into digital signals, and transmits the digital signals to the central control unit 100.

The switch unit 160 includes a switch for performing input to select operational modes, such as a preliminary scan mode and a scan mode. The switch unit 160 according to a preferred embodiment of the present invention enables an operational mode, depending on input time or input form, to be determined using a single switch. In addition, another embodiment of the switch unit 160 of the present invention may be configured to be provided with a plurality of buttons, and allow different buttons to be assigned to respective operational modes.

The central control unit 100 determines an operational mode based on a signal input through the switch unit. Thereafter, when the preliminary scan mode is determined, an operation is performed in the preliminary scan mode. In contrast, when the scan mode is determined, an operation is performed in the scan mode.

The operation in the preliminary scan mode of the ultrasonic diagnosis apparatus according to the present invention is described below.

When the preliminary scan mode is selected, the central control unit transmits a drive control signal for sequentially rotating the second stepping motor to the drive control unit, and the drive control unit rotates the second stepping motor in a yz direction (that is, a second direction) in response to the drive control signal received from the central control unit. As the second stepping motor rotates, the transducer also rotates. The transducer acquires the pieces of ultrasonic information of n scan lines in the yz direction while rotating in the yz direction. Meanwhile, the central control unit receives the pieces of ultrasonic information of n scan lines in the yz direction from the transducer, extracts a bladder image for a corresponding plane in the yz direction from the pieces of received ultrasonic information, and outputs the extracted image to the display unit. In this case, in the state in which the transducer is disposed on the abdomen of a patient and is oriented toward his or her urinary bladder in the preliminary scan mode, the scanning apparatus according to the present invention rotates in left and right directions relative to the patient, that is, a lateral direction with respect to the patient, and thus a two-dimensional image obtained as a result of the rotation is output to the display unit.

A user, who uses the scanning apparatus according to the present invention, causes the scanning apparatus to operate in the preliminary scan mode and then views the image output to the display unit, so that he or she can be quickly and accurately made aware of the location of the urinary bladder which is to be examined.

Furthermore, in the preliminary scan mode, the above-described process is periodically repeated until the scan mode is input and a two-dimensional image for a corresponding plane is output to the display unit. In this case, it is preferred that the repetition period be less than about 5 seconds.

Meanwhile, in another embodiment of the ultrasonic diagnosis apparatus according to the present invention for the preliminary scan mode, when the preliminary scan mode is selected, respective two-dimensional images for three planes are acquired, and are displayed on a single screen. In this case, it is preferred that the acquired three planes for two-dimensional images be formed to have different angles.

The operation of the ultrasonic diagnosis apparatus according to the present invention in the scan mode is described below.

When the scan mode is selected, the central control unit 100 rotates the first stepping motor and the second stepping motor, and thus the transducer acquires the pieces of ultrasonic information of n scan lines for each of m planes. A process of the transducer acquiring the pieces of ultrasonic information of n scan lines for each of m planes is as follows.

First, after the first stepping motor is fixed, the transducer acquires the ultrasonic information of a single scan line at a location to which movement is made while the second stepping motor is sequentially rotated n times by a predetermined angle, and thus the pieces of ultrasonic information of n scan lines for a single plane are acquired.

Thereafter, the above-described process (that is, the process of the transducer acquiring the pieces of ultrasonic information of n scan lines for a single plane at the corresponding location) is repeated while the first stepping motor, which moves in a direction orthogonal to the second stepping motor, is sequentially rotated m times by a predetermined angle, and thus the pieces of ultrasonic information of n scan lines for m planes, to which movement is made by the second stepping motor, are acquired.

The first stepping motor and the second stepping motor are rotated as described above, so that ultrasonic waves are emitted and received in the form of a cone, the vertex of which is formed by the transducer, therefore the three-dimensional volume of the urinary bladder can be measured.

Meanwhile, the central control unit 100 receives the pieces of ultrasonic information, which are acquired by the transducer, from the transducer through the analog signal processing unit 150. The central control unit 100 calculates the volume of urine in the urinary bladder, which is an examination object, using the signals transmitted from the analog signal processing unit 150, and outputs the ultrasonic image of the urinary bladder, which is an image related to the specific plane of the urinary bladder, to the display unit 170. The display unit 170 displays the image, which is transmitted from the central control unit, on the screen along with the volume of urine remaining in the urinary bladder.

As shown in FIG. 2, a rotational support 122 is connected to the first stepping motor 120. A second stepping motor 130 is mounted on the rotational support 122 and rotates along with the rotational support 122. The second stepping motor 130 is connected with a transducer support including a rotational axis. A transducer 110 is installed in the transducer support.

The central control unit 100 transmits drive control signals to the drive control unit 140 in response to an operational mode signal received from the switch unit 160, and the drive control unit 140 controls the motion of the first and second stepping motors 120 and 130 in response to the drive control signals, so that the ultrasonic image of the urinary bladder can be captured through the rotation of the transducer 110.

The second stepping motor 130 rotates by the predetermined angle in an yz plane, and the rotational axis 132 and the transducer support 134, which are connected to the second stepping motor via a gear, are rotated by the second stepping motor 130. Consequently, the transducer 110 installed in the transducer support 134 rotates in the second direction (that is, the yz plane).

Meanwhile, the rotational support 122, on which the second stepping motor 130 is mounted, is connected to the first stepping motor 120, so that the rotational support 122 also moves by the predetermined angle in a first direction (that is, an xy direction) as the first stepping motor 120 moves in an xy plane. Accordingly, the second direction, which is the direction in which the second stepping motor rotates, and the first direction, which is the direction in which the first stepping motor rotates, are orthogonal to each other.

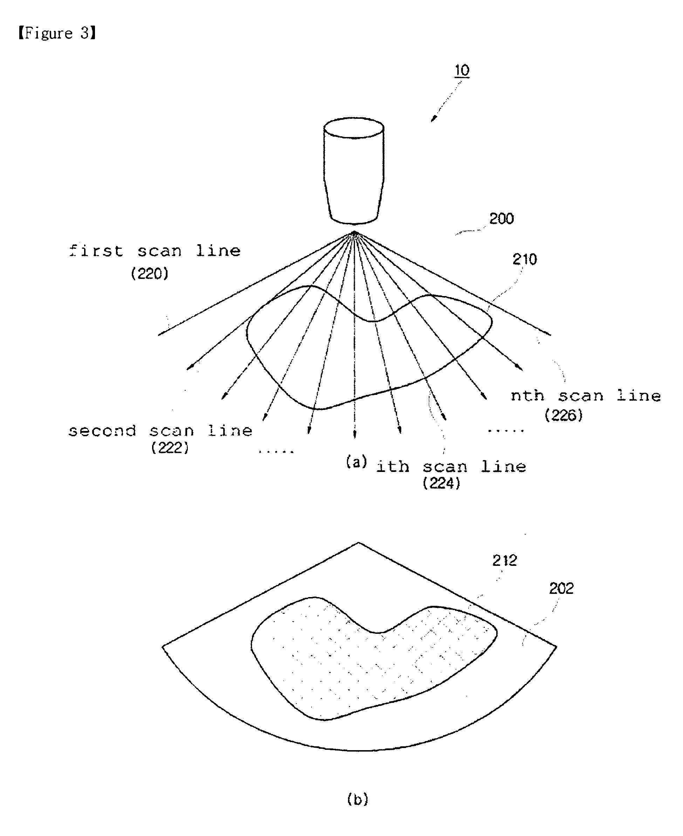

FIGS. 3(a) and 3(b) are diagrams illustrating a process of the ultrasonic diagnosis apparatus 10, according to the present invention, acquiring a bladder image for a single plane.

With reference to FIG. 3(a), in the ultrasonic diagnosis apparatus 10 in which the transducer is disposed on an arbitrary location of an abdomen 200 over the urinary bladder 210 of a patient, the central control unit causes the first stepping motor and the second stepping motor to be fixed, and detects ultrasonic signals at the corresponding location. Thereafter, a process of detecting ultrasonic signals at a corresponding angle while moving the second stepping motor by the predetermined angle in the yz direction is repeated, and thus ultrasonic signals for n scan lines, that is, a first scan line 220, a second scan line 222, · · · , ith scan line 224, · · · , nth scan line 226 are sequentially detected. After detecting n ultrasonic signals, the central control unit 100, as shown in FIG. 3(b), generates a two-dimensional image by processing ultrasonic signals for a corresponding plane, and displays the generated two-dimensional image on the display unit 170. FIG. 3(b) is a diagram showing the two-dimensional image output to the display unit 170, in which urine 212 in the urinary bladder 210 is displayed while being separated from organs 202 around the urinary bladder 210.

Meanwhile, the above-described process is repeated while the first stepping motor is rotated by the predetermined angle and, thus, ultrasonic signals for the n scan lines for the m planes are detected. As described above, a three-dimensional image is generated using two-dimensional images acquired for the m planes. In this case, it is preferred that the number m of the acquired two-dimensional images be equal to or greater than 4 and equal to and less than 30.

[Bladder volume measurement method]

A method of the central control unit 100 of the ultrasonic diagnosis apparatus 10 according to the preferred embodiment of the present invention, having the above-described construction, measuring the amount of urine in the urinary bladder using ultrasonic signals, is described below.

First, the central control unit determines whether an operational mode, which is input through the switch unit, is the preliminary scan mode or the scan mode at step 400.

If it is determined that the operational mode is the preliminary scan mode, pieces of ultrasonic information, which are obtained by scanning n scan lines for a single plane at a current location, are received at step 410. Thereafter, a two-dimensional bladder image for the corresponding plane is extracted from the pieces of received ultrasonic information, and is output to the display unit, at step 412. Accordingly, the user, who manipulates the ultrasonic diagnosis apparatus according to the present invention the present invention, causes the ultrasonic diagnosis apparatus to operate in the preliminary scan mode, and moves a probe or adjusts the tilt angle of the probe while viewing the two-dimensional image displayed on the screen, so that the urinary bladder can be located in the center portion of the ultrasonic image and, in addition, the location and tilt angle of the probe can be detected such that a large bladder plane is viewed. From the above-described process, an operation can be performed in the scan mode at a location close to the center of the urinary bladder, and, as a result, the measurement of the urinary bladder can be accurately and quickly performed.

If it is determined that the operational mode is the scan mode, pieces of ultrasonic information, which are obtained by scanning the urinary bladder, which is an object to be examined, along n scan lines for each of m planes, are received from the transducer of the ultrasonic diagnosis apparatus at step 420. The process of receiving pieces of ultrasonic information of n scan lines for a single plane is repeatedly performed on the m planes, and thus the pieces of ultrasonic information of each of n scan lines for m plans are received. The number of planes to be scanned and the number of scan lines for a single plane may be determined according to the region and size of the object to be examined. In the case of measuring the urinary bladder, the number of scan lines and the number of images may be determined such that the entire region of the urinary bladder can be included. For example, in the case of scanning the urinary bladder, the entire region of the urinary bladder can be sufficiently included using about 67 lines if the angle between lines for forming a single image is 1.8°.

Thereafter, the locations of front and rear walls are detected from pieces of ultrasonic information of scan lines constituting each plane at step S421, and difference values Depth[1], Depth[2], · · · , Depth[n] corresponding to the differences between the locations of the detected front and rear walls for the respective scan lines are obtained at step S422. Thereafter, the area of the corresponding plane is obtained by summing the difference values for the scan lines constituting each plane.

The above-described process of obtaining the area of each plane is repeatedly performed on m planes, and thus the areas Area[1], Area[2], · · · · , Area[m] of the respective planes are obtained at step 424. In this case, the method of obtaining the area of each plane using difference values corresponding to the differences between the locations of the front and rear walls of the urinary bladder for the respective scan lines may be implemented in various ways. As an example, the entire area of each plane may be obtained by obtaining an area for a sector for a single scan line using the rotational angle of the second stepping motor 130 and summing sector areas for respective lines having rear walls. As another example, the entire area may be obtained by summing trapezoidal areas, which are obtained by repeating a process of obtaining an area for a trapezoid, which is formed by the two front walls and two rear walls of two neighboring scan lines.

Meanwhile, if scanning is performed in a state in which the center of a first rotational axis moves from the center of the urinary bladder when a three-dimensional volume is obtained using a plurality of two-dimensional images, an amount smaller than an actual amount is calculated and, thus, an error relative to the actual amount is generated. Accordingly, numerical correction is performed to reduce such error and accurately measure the amount of urine in the urinary bladder. The process of performing the numerical correction is described below.

First, difference values corresponding to the differences between the locations of front and rear walls of the urinary bladder for n scan lines constituting each plane are obtained. Thereafter, the maximum difference values bladderDepth[1], bladderDepth[2], · · · , bladderDepth[m] of the respective planes are obtained among the difference values at step 426, and the greatest 'MaxbladderDepth' of the maximum difference values of the respective planes is obtained at step S428.

Thereafter, at step 430, the correction coefficients ComFactor[1], ComFactor[2], · · · , ComFactor[i], and Com-Factor[m] for the respective planes are obtained using the greatest 'MaxBladderDepth' of the maximum difference values and the maximum difference values BladderDepth[1], BladderDepth[2], · · · , BladderDepth[m] of the respective planes, based on the following Equation 1.

[Equation 1]

$$ComFactor[i] = \frac{MaxBladderDepth}{BladderDepth[i]}$$

Thereafter, given the assumption that a bladder image for each plane is a circle, radii r[1], r[2], · · · , r[i], and r[m] of respective circles having the same areas as the areas Area[1], Area[2], · · · , Area[m] of the respective planes are obtained and are determined to be radii for bladder images of the respective planes at step S432.

Thereafter, at step S434, corrected radii ComR[1], ComR[2], · · · , ComR[i], and ComR[m] with respect to the correction coefficients and the radii for the urinary bladder images of the respective planes are obtained using the following Equation 2:

[Equation 2]

$$ComR[i] = ComFactor[i] \times r[i]$$

An average radius 'AverageR', which is the average value of the calculated corrected radii for the images of the respective planes, is obtained at step S436. Thereafter, given the assumption that the complete bladder is a sphere, the total volume V of urine in the urinary bladder is obtained by applying the average radius to the following Equation 3 at step S438.

$$[\text{Equation 3}]$$

$$V = \frac{4}{3}\pi AverageR^3$$

From the above-described process, the ultrasonic diagnosis apparatus for the urinary bladder according to the present invention can accurately detect the amount of urine in the urinary bladder.

Furthermore, the ultrasonic diagnosis apparatus for the urinary bladder according to the present invention can extract pieces of bladder information, such as the thickness and weight of the urinary bladder, as well as information about the amount of urine remaining in the urinary bladder, from two-dimensional images, and can output the pieces of extracted information of the urinary bladder to the display unit.

Although the present invention has been described in detail in conjunction with the preferred embodiment, the present invention is described only for illustrative purposes, and is not limited thereto. Those skilled in the art will appreciate that various modifications and applications, which are not described above, are possible within a range that does not change the substantial characteristics of the present invention. For example, in the present embodiment, the method of obtaining the area of a corresponding plane using the rotational angles of the first stepping motor and the second stepping motor and ultrasonic information about the respective scan lines may be modified and implemented in various ways to improve scanning performance. Furthermore, it should be appreciated that the differences regarding the modifications and the applications are included in the scope of the present invention, which is defined by the accompanying claims.

[Industrial Applicability]

The ultrasonic diagnosis apparatus and method according to the present invention may be widely used in the medical field.

**Claims**

1. An ultrasonic diagnosis apparatus (10) for a urinary bladder, comprising:

   a transducer (110) for emitting ultrasonic signals and receiving ultrasonic signals reflected from an object;
   a transducer support (122) configured such that the transducer is fixedly installed therein;
   an analog signal processing unit (150) for converting the ultrasonic signals, which are transmitted from the transducer, into digital signals;
   a display unit (170) for outputting specific image signals;
   a central control unit (100) for performing image processing on the digital signals from the analog signal processing unit (150), outputting results of the processing to the display unit (170), and controlling overall operation of the apparatus (10);
   a first stepping motor (120) for rotating the transducer in a first direction;
   a second stepping motor (130) for rotating the transducer in a second direction;
   a drive control unit (140) for controlling operation of the first and second stepping motors in response to drive control signals provided by the central control unit (100); and
   a switch unit (160) for selecting operation modes;
   wherein, when a first operational mode is selected by the switch unit (160), the central control unit (100) is configured to receive pieces of ultrasonic information of n scan lines (220-226) for a single plane at a current location from the transducer (101), acquires an image from the recieved pieces of ultrasonic information, and output the acquired image to the display unit (170), and
   when a second operational mode is selected by the switch unit (160), the central control unit (100) is configured to receive pieces of ultrasonic information of n scan lines for each of m planes from the transducer (110) and calculate volume information, about the urinary bladder using the received pieces of ultrasonic information of n scan lines for each of a planes,

wherein, when the second operational mode is selected, the central control unit (100) is configured to fix the first stepping motor (120) and acquire ultrasonic information while sequentially rotating the second stepping motor (130) n times by a predetermined angle, thus acquiring the pieces of ultrasonic information of n scan lines for a single plane, and the central control unit (100) is configured to acquire the pieces of ultrasonic information of n scan lines for each of m planes by repeating the acquisition of the pieces of ultrasonic information of n scan lines for a single plane m times while sequentially rotating the first stepping motor (120) by the predetermined angle,

2. The ultrasonic diagnosis apparatus according to claim 1, wherein, when the first operational mode is selected, the central control unit (100) is configured to transmit a drive control signal for rotating the second stepping motor (130) at the current location to the drive control unit (140).

the drive control unit (140) is configured to sequentially rotate the second stepping motor (130) in response to the drive control signal received from the central control unit (100), and

the central control unit (100) is configured to receive the pieces of ultrasonic information of n scan lines for a single plane from the transduce (110) as rotated by the second stepping motor, extract, a two-dimensional bladder image for the single plane from the received pieces of ultrasonic information, and output the extracted two-dimensional bladder image to the display unit (170).

**characterised in that** the central control unit (100) is configured to calculate the volume information by detecting locations of front and rear walls of the urinary bladder for the respective scan lines, obtaining difference values corresponding to differences between the detected locations of the front and rear walls for the respective scan lines, obtaining areas for urinary bladder images of the respective planes using the difference values for the n scan lines constituting each plane, obtaining correction coefficients for the respective planes, calculating radii of respective circles having areas identical to the areas for the urinary bladder images of the respective planes, calculating corrected radii for the respective planes by applying the correction coefficients to the calculated radii for the respective planes, obtaining an average radius of the corrected radii for the respective planes, and obtaining a volume of a sphere using the average radius, which volume is the volume of urine in the urinary bladder.

3. The ultrasonic diagnosis apparatus according to claim 1, wherein the central control unit (100) is configured to detect a maximum of the difference values for the respective scan lines for each plane, obtain a greatest of the maximum values for the respective planes, and obtain the correction coefficients for the respective planes using ratios of the maximum values for the respective planes to the greatest of the maximum values.

4. An ultrasonic diagnosis method for measuring volume information about a urinary bladder using an ultrasonic diagnosis apparatus (10), comprising the steps of:

a) determining an operational mode input from an outside;

b) if it is determined that the operational mode input from the outside is a preliminary scan mode, receiving pieces of ultrasonic information of n scan lines (220-226) for a single plane at a current location from a transducer (110), extracting a urinary bladder image for the single plane from the received pieces of ultrasonic information, and outputting the extracted urinary bladder image to a display unit (170); and

c) if it is determined that the operational mode input from the outside is a scan mode, sequentially receiving pieces of ultrasonic information of n scan lines for each of m planes from the transducer, and detecting the volume information using the sequentially received pieces of ultrasonic information,

**characterised in that** step c) comprises the steps of:

c1) detecting (S421) locations of front and rear walls of the urinary bladder for the respective scan lines;

c2) obtaining (S422) difference values between the detected locations of the front and rear walls for the respective scan lines;

c3) obtaining (S424) areas for urinary bladder images of the respective planes using the difference values for the scan lines of each plane;

c4) obtaining (S430) correction coefficients for the respective planes;

c5) calculating radii (S432) respective circles having areas identical to areas for the urinary bladder images of the respective planes, and calculating corrected radii by applying the correction coefficients for the respective planes to the radii for the respective planes;

c6) obtaining (S436) an average radius of the corrected radii for the respective planes; and

g) obtaining (S438) a volume of a sphere using the average radius, which volume is the volume of urine in the urinary bladder.

5. The ultrasonic diagnosis method according to claim 4, wherein the step c4) comprises the steps of:

   1) detecting (S426) a maximum of the difference values for the respective scan lines for each plane;
   2) obtaining (S428) a greatest of the maximum values for the respective planes; and
   3) obtaining correction coefficients for the respective planes using ratios of the maximum values for the respective planes to the greatest of the maximum values.

6. The ultrasonic diagnosis method according to claim 5, wherein the corrected coefficients of the step 3) are calculated using the following Equation :

$$ComFactor[i] = \frac{MaxBladderDepth}{BladderDepth[i]}$$

where ComFactor[i] is a corrected coefficient for an ith plane, bladderDepth[i] is a maximum of the difference values between locations of front and rear walls for scan lines for the ith plane, and MaxBladderDepth is a greatest of maximum values of the respective planes.

7. The ultrasonic diagnosis method according to claim 4, wherein urinary bladder information detected by the ultrasonic diagnostic method includes at least one of a thickness of the urinary bladder and a weight of the urinary bladder.

8. The ultrasonic diagnosis method according to claim 4, wherein the urinary bladder image in step 6) is two-dimensional, the step c) comprises extracting m two-dimensional images from the sequentially received pieces of ultrasonic information, detecting urinary bladder information including the volume information from the extracted m two-dimensional images, and outputting the detected urinary bladder information to the display unit (170), and the step b) is periodically performed at regular intervals until the scan mode is selected as the operational mode input from the outside.

9. The ultrasonic, diagnosis method according to claim 8, wherein, at the step c), the urinary bladder information includes at least one of a thickness of the urinary bladder and a weight of the urinary bladder.

10. The ultrasonic diagnosis method according to any of claims 4 to 8. wherein the number m is equal to or greater than 4 and equal to and less than 30.

11. The ultrasonic diagnosis method according to claim 8, wherein the repetition period of the step b) is less than 5 seconds.

12. The ultrasonic diagnosis method according to any of claims 4 to 8, wherein the urinary bladder image extrated in the preliminary scan mode of the step 6) is a lateral image acquired through scanning in a lateral direction of a patient using the transducer direction.

13. The ultrasonic diagnosis method according to any of claims 4 to 8, wherein the preliminary scan mode of the step b) allows two-dimensional images for a maximum of three planes to be acquired, and allows the acquired images to be displayed on a single screen.

**Patentansprüche**

1. Eine Ultraschall-Diagnosevorrichtung (10) für eine Harnblase, aufweisend:

   einen Wandler (110) zum Ausgeben von Ultraschallsignalen und zum Empfangen von von einem Objekt reflektierten Ultraschallsignalen,
   eine Wandlerhalterung (122), die derart konfiguriert ist, dass der Wandler fest darin installiert ist,
   eine Analogsignal-Verarbeitungseinheit (150) zum Umwandeln der von dem Wandler übermittelten Ultraschallsignale in digitale Signale,

eine Anzeigeeinheit (170) zum Ausgeben von spezifischen Bildsignalen,

eine zentrale Steuereinheit (100) zum Durchführen einer Bildverarbeitung an den digitalen Signalen aus der Analogsignal-Verarbeitungseinheit (150), zum Ausgeben der Ergebnisse der Verarbeitung an die Anzeigeeinheit (170) und zum Steuern der Gesamtfunktion der Vorrichtung (10),

einen ersten Schrittmotor (120) zum Drehen des Wandlers in einer ersten Richtung,

einen zweiten Schrittmotor (130) zum Drehen des Wandlers in einer zweiten Richtung,

eine Antriebssteuereinheit (10) zum Steuern der Funktion des ersten und des zweiten Schrittmotors in Antwort auf von der zentralen Steuereinheit (100) bereitgestellte Antriebssteuersignale, und

eine Umschalteinheit (160) zum Auswählen von Betriebsmodi,

wobei, wenn mittels der Umschalteinheit (160) ein erster Betriebsmodus ausgewählt wird, die zentrale Steuereinheit (100) konfiguriert ist zum Empfangen von Teilen von Ultraschallinformation von n Abtastzeilen (220 bis 226) für eine einzige Ebene an einer aktuellen Position von dem Wandler (110), zum Erfassen eines Bildes aus den empfangenen Teilen von Ultraschallinformation und zum Ausgeben des erfassten Bildes an die Anzeigeeinheit (170), und wobei,

wenn mittels der Umschalteinheit (160) ein zweiter Betriebsmodus ausgewählt wird, die zentrale Steuereinheit (100) konfiguriert ist zum Empfangen von Teilen von Ultraschallinformation von n Abtastzeilen für jede von m Ebenen von dem Wandler (110) und zum Berechnen von Volumeninformationen über die Harnblase mittels der empfangenen Teile von Ultraschallinformation von n Abtastzeilen für jede von m Ebenen,

wobei, wenn der zweite Betriebsmodus ausgewählt ist, die zentrale Steuereinheit (100) zum Fixieren des ersten Schrittmotors (120) und zum Erfassen von Ultraschallinformationen konfiguriert ist, während der zweite Schrittmotor (130) n Mal sequentiell um einen vorbestimmten Winkel gedreht wird, wodurch die Teile von Ultraschallinformation von n Abtastzeilen für eine einzige Ebene erfasst werden, und

die zentrale Steuereinheit (100) zum Erfassen der Teile von Ultraschallinformation von n Abtastlinien für jede von m Ebenen konfiguriert ist, durch das m-malige Wiederholen des Erfassens der Teile von Ultraschallinformationen von n Abtastzeilen für eine einzige Ebene, während der erste Schrittmotor (120) um den vorbestimmten Winkel gedreht wird,

**dadurch gekennzeichnet, dass** die zentrale Steuereinheit (100) konfiguriert ist zum Berechnen der Volumeninformation durch Detektieren von Positionen der Vorderwand und der Rückwand der Harnblase für die jeweiligen Abtastzeilen, zum Erhalten von Differenzwerten, die Differenzen zwischen den detektierten Positionen der Vorderwand und der Rückwand für die jeweiligen Abtastzeilen entsprechen, zum Erhalten von Flächen für Harnblasenbilder der jeweiligen Ebenen mittels der Differenzwerte für die n Abtastzeilen, die jede Ebene bilden, zum Erhalten von Korrekturkoeffizienten für die jeweiligen Ebenen, zum Berechnen von Radien jeweiliger Kreise, deren Flächen mit den Flächen für die Harnblasenbilder der jeweiligen Ebenen identisch sind, zum Berechnen von korrigierten Radien für die jeweiligen Ebenen durch Anwenden der Korrekturkoeffizienten auf die berechneten Radien für die jeweiligen Ebenen, zum Erhalten eines Durchschnittsradius der korrigierten Radien für die jeweiligen Ebenen, und zum Erhalten eines Volumens einer Sphäre mittels des Durchschnittsradius, wobei das Volumen das Urinvolumen in der Harnblase ist.

2. Die Ultraschall-Diagnosevorrichtung gemäß Anspruch 1, wobei, wenn der erste Betriebsmodus ausgewählt ist, die zentrale Steuereinheit (100) zum Übermitteln eines Antriebssteuersignals zum Drehen des zweiten Schrittmotors (130) an der aktuellen Position zu der Antriebssteuereinheit (140) konfiguriert ist,

die Antriebssteuereinheit (140) zum sequentiellen Drehen des zweiten Schrittmotors (130) in Antwort auf das von der zentralen Steuereinheit (100) empfangene Antriebssteuersignal konfiguriert ist, und

die zentrale Steuereinheit (100) konfiguriert ist zum Empfangen der Teile von Ultraschallinformation von n Abtastzeilen für eine einzige Ebene von dem Wandler (110), wenn dieser mittels des zweiten Schrittmotors gedreht wird, zum Extrahieren eines zweidimensionalen Blasenbildes für die einzige Ebene aus den empfangenen Teilen von Ultraschallinformation, und zum Ausgeben des extrahierten zweidimensionalen Blasenbildes an die Anzeigeeinheit (170).

3. Die Ultraschall-Diagnosevorrichtung gemäß Anspruch 1, wobei die zentrale Steuereinheit (100) konfiguriert ist zum Detektieren eines Maximums der Differenzwerte für die jeweiligen Abtastzeilen für jede Ebene, zum Erhalten eines größten der Maximalwerte für die jeweiligen Ebenen, und zum Erhalten der Korrekturkoeffizienten für die jeweiligen Ebenen mittels Verhältnissen der Maximalwerte für die jeweiligen Ebenen zu dem größten der Maximalwerte.

4. Ein Ultraschall-Diagnoseverfahren zum Messen der Volumeninformation über eine Harnblase mittels einer Ultraschall-Diagnosevorrichtung (10), die folgenden Schritte aufweisend:

a) Ermitteln eines von außen eingegebenen Betriebsmodus,

b) wenn ermittelt wird, dass der von außen eingegebene Betriebsmodus ein vorläufiger Abtastmodus ist, Empfangen von Teilen von Ultraschallinformationen von n Abtastzeilen (220 bis 226) für eine einzige Ebene an einer aktuellen Position von einem Wandler (110), Extrahieren eines Harnblasenbildes für die einzige Ebene aus den empfangenen Teilen von Ultraschallinformation und Ausgeben des extrahierten Harnblasenbildes an eine Anzeigeeinheit (170), und

c) wenn ermittelt wird, dass der von außen eingegebene Betriebsmodus ein Abtastmodus ist, sequentielles Empfangen von Teilen von Ultraschallinformation von n Abtastzeilen für jede von m Ebenen von dem Wandler und Detektieren der Volumeninformation mittels der sequentiell empfangenen Teile von Ultraschallinformation, **dadurch gekennzeichnet, dass** der Schritt c) die folgenden Schritte aufweist:

c1) Detektieren (S421) von Positionen der Vorderwand und der Rückwand der Harnblase für die jeweiligen Abtastzeilen,
c2) Erhalten (S422) von Differenzwerten zwischen den detektierten Positionen der Vorderwand und der Rückwand für die jeweiligen Abtastzeilen,
c3) Erhalten (S424) von Flächen für Harnblasenbilder der jeweiligen Ebenen mittels der Differenzwerte für die Abtastzeilen jeder Ebene,
c4) Erhalten (S430) von Korrekturkoeffizienten für die jeweiligen Ebenen,
c5) Berechnen von Radien (S432) von jeweiligen Kreisen, deren Flächen mit Flächen für die Harnblasenbilder der jeweiligen Ebenen identisch sind, und Berechnen von korrigierten Radien durch Anwenden der Korrekturkoeffizienten für die jeweiligen Ebenen auf die Radien für die jeweiligen Ebenen,
c6) Erhalten (S436) eines Durchschnittsradius der korrigierten Radien für die jeweiligen Ebenen, und

g) Erhalten (S438) eines Volumens einer Sphäre mittels des Durchschnittsradius, wobei das Volumen das Urinvolumen in der Harnblase ist.

5. Das Ultraschall-Diagnoseverfahren gemäß Anspruch 4, wobei Schritt c4) die folgenden Schritte aufweist:

1) Detektieren (S426) eines Maximums der Differenzwerte für die jeweiligen Abtastzeilen für jede Ebene,
2) Erhalten (S428) eines größten der Maximalwerte für die jeweiligen Ebenen, und
3) Erhalten von Korrekturkoeffizienten für die jeweiligen Ebenen mittels Verhältnissen der Maximalwerte für die jeweiligen Ebenen zu den größten der Maximalwerte.

6. Das Ultraschall-Diagnoseverfahren gemäß Anspruch 5, wobei die Korrekturkoeffizienten aus Schritt 3) mittels der folgenden Gleichung berechnet werden:

$$ComFaktor\,[i] = \frac{MaxBlasentiefe}{Blasentiefe\,[i]}\,,$$

wobei der ComFaktor [i] ein Korrekturkoeffizient für eine i-te Ebene ist, die Blasentiefe [i] ein Maximum der Differenzwerte zwischen Positionen der Vorderwand und der Rückwand für Abtastzeilen für die i-te Ebene ist und MaxBlasentiefe ein größter von Maximalwerten der jeweiligen Ebenen ist.

7. Das Ultraschall-Diagnoseverfahren gemäß Anspruch 4, wobei die mittels des Ultraschall-Diagnoseverfahrens detektierte Harnblaseninformation mindestens eines von einer Dicke der Harnblase und einem Gewicht der Harnblase enthält.

8. Das Ultraschall-Diagnoseverfahren gemäß Anspruch 4, wobei das Harnblasenbild in Schritt b) zweidimensional ist, wobei der Schritt c) aufweist: das Extrahieren von m zweidimensionalen Bildern aus den sequentiell empfangenen Teilen von Ultraschallinformation, das Detektieren von Harnblaseninformation einschließlich der Volumeninformation aus den extrahierten m zweidimensionalen Bildern und das Ausgeben der detektierten Harnblaseninformation an die Anzeigeeinheit (170),
und wobei der Schritt b) in regelmäßigen Intervallen periodisch durchgeführt wird, bis der Abtastmodus als der von außen eingegebene Betriebsmodus ausgewählt ist.

9. Das Ultraschall-Diagnoseverfahren gemäß Anspruch 8, wobei in Schritt c) die Harnblaseninformation mindestens eines von einer Dicke der Harnblase und einem Gewicht der Harnblase aufweist.

10. Das Ultraschall-Diagnoseverfahren gemäß einem der Ansprüche 4 bis 8, wobei die Anzahl m größer oder gleich 4 und kleiner oder gleich 30 ist.

11. Das Ultraschall-Diagnoseverfahren gemäß Anspruch 8, wobei die Wiederholungsperiode des Schrittes b) weniger als 5 Sekunden beträgt.

12. Das Ultraschall-Diagnoseverfahren gemäß einem der Ansprüche 4 bis 8, wobei das in dem vorläufigen Abtastmodus des Schrittes b) extrahierte Harnblasenbild ein durch Abtasten in einer seitlichen Richtung eines Patienten unter Verwendung der Wandlerrichtung erfasstes seitliches Bild ist.

13. Das Ultraschall-Diagnoseverfahren gemäß einem der Ansprüche 4 bis 8, wobei der vorläufige Abtastmodus des Schrittes b) das Erfassen von zweidimensionalen Bildern für ein Maximum von drei Ebenen ermöglicht und ermöglicht, dass die erfassten Bilder auf einem einzigen Bildschirm angezeigt werden.

**Revendications**

1. Appareil de diagnostic par ultrasons (10) pour une vessie, comprenant :

un transducteur (110) pour émettre des signaux ultrasonores et recevoir des signaux ultrasonores réfléchis par un objet ;
un support de transducteur (122) configuré de sorte que le transducteur soit installé fixement dans celui-ci ;
une unité de traitement de signaux analogiques (150) pour convertir les signaux ultrasonores, qui sont émis par le transducteur, en des signaux numériques ;
une unité d'affichage (170) pour délivrer des signaux d'image spécifiques;
une unité de commande centrale (100) pour appliquer un traitement d'image aux signaux numériques provenant de l'unité de traitement de signaux analogiques (150), délivrer les résultats du traitement à l'unité d'affichage (170) et commander le fonctionnement global de l'appareil (10) ;
un premier moteur pas à pas (120) pour faire tourner le transducteur dans une première direction;
un deuxième moteur pas à pas (130) pour faire tourner le transducteur dans une deuxième direction ;
une unité de commande d'entraînement (140) pour commander le fonctionnement des premier et deuxième moteur pas à pas en réponse à des signaux de commande d'entraînement fournis par l'unité de commande centrale (100) ; et
une unité de commutation (160) pour sélectionner des modes de fonctionnement ;
dans lequel, lorsqu'un premier mode de fonctionnement est sélectionné par l'unité de commutation (160), l'unité de commande centrale (100) est configurée pour recevoir, du transducteur (110), des éléments d'informations ultrasonores de n lignes de balayage (220 à 226) pour un plan unique à un emplacement actuel, acquérir une image à partir des éléments d'informations ultrasonores reçus, et délivrer l'image acquise à l'unité d'affichage (170), et
lorsqu'un deuxième mode de fonctionnement est sélectionné par l'unité de commutation (160), l'unité de commande centrale (100) est configurée pour recevoir, du transducteur (110), des éléments d'informations ultrasonores de n lignes de balayage pour chacun de m plans, et calculer des informations de volume concernant la vessie en utilisant les éléments d'informations ultrasonores reçus de n lignes de balayage pour chacun de m plans,
dans lequel, lorsque le deuxième mode de fonctionnement est sélectionné, l'unité de commande centrale (100) est configurée pour arrêter le premier moteur pas à pas (120) et acquérir des informations ultrasonores tout en faisant tourner séquentiellement le deuxième moteur pas à pas (130) n fois d'un angle prédéterminé, acquérant ainsi les éléments d'informations ultrasonores de n lignes de balayage pour un plan unique, et
l'unité de commande centrale (100) est configurée pour acquérir les éléments d'informations ultrasonores de n lignes de balayage pour chacun de m plans en répétant l'acquisition des éléments d'informations ultrasonores de n lignes de balayage pour un plan unique m fois tout en faisant tourner séquentiellement le premier moteur pas à pas (120) de l'angle prédéterminé,
**caractérisé en ce que** l'unité de commande centrale (100) est configurée pour calculer les informations de volume en détectant les emplacements des parois avant et arrière de la vessie pour les lignes de balayage respectives, en obtenant des valeurs de différence correspondant aux différences entre les emplacements détectés des parois avant et arrière pour les lignes de balayage respectives, en obtenant des aires pour les images de vessie des plans respectifs en utilisant les valeurs de différence pour les n lignes de balayage constituant chaque plan, en obtenant des coefficients de correction pour les plans respectifs, en calculant les rayons de cercles respectifs ayant des aires identiques aux aires pour les images de vessie des plans respectifs, en calculant des rayons corrigés pour les plans respectifs en appliquant les coefficients de correction aux rayons calculés pour les plans respectifs, en obtenant un rayon moyen des rayons corrigés pour les plans respectifs, et en obtenant un volume d'une sphère en utilisant le rayon moyen, lequel volume est le volume d'urine dans la vessie.

2. Appareil de diagnostic par ultrasons selon la revendication 1, dans lequel, lorsque le premier mode de fonctionnement est sélectionné, l'unité de commande centrale (100) est configurée pour transmettre un signal de commande d'entraînement pour faire tourner le deuxième moteur pas à pas (130) à l'emplacement actuel à l'unité de commande d'entraînement (140),
l'unité de commande d'entraînement (140) est configurée pour faire tourner séquentiellement le deuxième moteur pas à pas (130) en réponse au signal de commande d'entraînement reçu de l'unité de commande centrale (100), et l'unité de commande centrale (100) est configurée pour recevoir les éléments d'informations ultrasonores de n lignes de balayage pour un plan unique du transducteur (110) alors qu'il est tourné par le deuxième moteur pas à pas, extraire une image bidimensionnelle de vessie pour le plan unique des éléments d'informations ultrasonores reçus, et délivrer l'image bidimensionnelle de vessie extraite à l'unité d'affichage (170).

3. Appareil de diagnostic par ultrasons selon la revendication 1, dans lequel l'unité de commande centrale (100) est configurée pour détecter un maximum des valeurs de différence pour les lignes de balayage respectives pour chaque plan, obtenir une plus grande des valeurs maximums pour les plans respectifs, et obtenir les coefficients de correction pour les plans respectifs en utilisant les rapports entre les valeurs maximums pour les plans respectifs et la plus grande des valeurs maximums.

4. Procédé de diagnostic par ultrasons pour mesurer des informations de volume concernant une vessie en utilisant un appareil de diagnostic par ultrasons (10), comprenant les étapes suivantes :

a) déterminer un mode de fonctionnement entré de l'extérieur ;
b) s'il est déterminé que le mode de fonctionnement entré de l'extérieur est un mode de balayage préliminaire, recevoir, d'un transducteur (110), des éléments d'informations ultrasonores de n lignes de balayage (220 à 226) pour un plan unique à un emplacement actuel, extraire une image de vessie pour le plan unique des éléments d'informations ultrasonores reçus, et délivrer l'image de vessie extraite à une unité d'affichage (170) ; et
c) s'il est déterminé que le mode de fonctionnement entré de l'extérieur est un mode de balayage, recevoir séquentiellement, du transducteur, des éléments d'informations ultrasonores de n lignes de balayage pour chacun de m plans, et détecter les informations de volume en utilisant les éléments d'informations ultrasonores reçus séquentiellement, **caractérisé en ce que** l'étape c) comprend les étapes consistant à :

c1) détecter (S421) les emplacements des parois avant et arrière de la vessie pour les lignes de balayage respectives ;
c2) obtenir (S422) des valeurs de différence entre les emplacements détectés des parois avant et arrière pour les lignes de balayage respectives ;
c3) obtenir (S424) des aires pour les images de vessie des plans respectifs en utilisant les valeurs de différence pour les lignes de balayage de chaque plan ;
c4) obtenir (S430) des coefficients de correction pour les plans respectifs;
c5) calculer les rayons (S432) de cercles respectifs ayant des aires identiques aux aires des images de vessie des plans respectifs, et calculer des rayons corrigés en appliquant les coefficients de correction pour les plans respectifs aux rayons pour les plans respectifs ;
c6) obtenir (S436) un rayon moyen des rayons corrigés pour les plans respectifs ; et

g) obtenir (S438) un volume d'une sphère en utilisant le rayon moyen, lequel volume est le volume d'urine dans la vessie.

5. Procédé de diagnostic par ultrasons selon la revendication 4, dans lequel l'étape c4) comprend les étapes suivantes :

1) détecter (S426) un maximum des valeurs de différence pour les lignes de balayage respectives pour chaque plan ;
2) obtenir (S428) une plus grande des valeurs maximums pour les plans respectifs ; et

3) obtenir des coefficients de correction pour les plans respectifs en utilisant les rapports entre les valeurs maximums pour les plans respectifs et la plus grande des valeurs maximums.

**6.** Procédé de diagnostic par ultrasons selon la revendication 5, dans lequel les coefficients corrigés de l'étape 3) sont calculés en utilisant l'équation suivante :

$$ComFactor[i] = \frac{MaxBladderDepth}{BladderDepth[i]}$$

où ComFactor[i] est un coefficient corrigé pour un $i^e$ plan, BladderDepth[i] est un maximum des valeurs de différence entre les emplacements des parois avant et arrière pour les lignes de balayage pour le $i^e$ plan, et MaxBladderDepth est une plus grande des valeurs maximums des plans respectifs.

**7.** Procédé de diagnostic par ultrasons selon la revendication 4, dans lequel les informations de vessie détectées par le procédé de diagnostic par ultrasons comprennent au moins l'un d'une épaisseur de la vessie et d'un poids de la vessie.

**8.** Procédé de diagnostic par ultrasons selon la revendication 4, dans lequel l'image de vessie de l'étape b) est bidimensionnelle,
l'étape c) consiste à extraire m images bidimensionnelles des éléments d'informations ultrasonores reçus séquentiellement, détecter des informations de vessie comprenant les informations de volume à partir des m images bidimensionnelles extraites, et délivrer les informations de vessie détectées à l'unité d'affichage (170),
et l'étape b) est effectuée périodiquement à des intervalles réguliers jusqu'à ce que le mode de balayage soit sélectionné en tant que mode de fonctionnement entré de l'extérieur.

**9.** Procédé de diagnostic par ultrasons selon la revendication 8, dans lequel, à l'étape c), les informations de vessie comprennent au moins l'un d'une épaisseur de la vessie et d'un poids de la vessie.

**10.** Procédé de diagnostic par ultrasons selon l'une quelconque des revendications 4 à 8, dans lequel le nombre m est égal ou supérieur à 4 et est égal ou inférieur à 30.

**11.** Procédé de diagnostic par ultrasons selon la revendication 8, dans lequel la période de répétition de l'étape b) est inférieure à 5 secondes.

**12.** Procédé de diagnostic par ultrasons selon l'une quelconque des revendications 4 à 8, dans lequel l'image de vessie extraite dans le mode de balayage préliminaire de l'étape b) est une image latérale acquise par balayage dans une direction latérale d'un patient en utilisant la direction de transducteur.

**13.** Procédé de diagnostic par ultrasons selon l'une quelconque des revendications 4 à 8, dans lequel le mode de balayage préliminaire de l'étape b) permet l'acquisition d'images bidimensionnelles pour trois plans au maximum, et permet l'affichage des images acquises sur un écran unique.

【Figure 1】

10

180 memory

110 — transducer

Analog Signal Processing unit — 150

First Stepping motor — 120

Drive Control unit — 140

Central Control unit — 100

Display unit — 170

Second Stepping motor — 130

Switch unit — 160

【Figure 2】

【Figure 3】

10

200

210

first scan line
(220)

second scan line
(222)

nth scan line
(226)

. . . . .

(a) ith scan line
(224)

. . . . .

212

202

(b)

【Figure 4】

```
                              ┌─────────┐
                              │  START  │
                              └────┬────┘
                                   │
┌─────────────────┐  YES           │
│ Display image    │◄──────◄ Operational mode =  ►────── S400
│ for single plane │       ◄ preliminary scan mode? ►
└────────┬─────────┘            │
         │                     NO
    S412    NO                  │
                         ◄ Operational mode = scan mode? ►──── S402
                                │
                               YES
                                │
         ┌──────────────────────────────────────────────┐
         │ receive pieces of ultrasonic information      │
         │ obtained by scanning urinary bladder along n  ├── S420
         │ scan lines for each of m planes               │
         └──────────────────┬───────────────────────────┘
                            │
         ┌──────────────────────────────────────────────┐
         │ detect locations of front and rear walls from │
         │ pieces of ultrasonic information of all of     ├── S421
         │ scan lines constituting each plane             │
         └──────────────────┬───────────────────────────┘
                            │
         ┌──────────────────────────────────────────────┐
         │ obtain difference values between detected      │
         │ locations of front and rear walls for          ├── S422
         │ respective scan lines of corresponding plane   │
         └──────────────────┬───────────────────────────┘
                            │
         ┌──────────────────────────────────────────────┐
         │ obtain areas for images of respective planes   │
         │ using difference values for scan lines         ├── S424
         │ constituting each plane                        │
         └──────────────────┬───────────────────────────┘
                            │
         ┌──────────────────────────────────────────────┐
         │ obtain maximum of difference values between    │
         │ front and rear walls for n scan lines          ├── S426
         │ constituting each plane                        │
         └──────────────────┬───────────────────────────┘
                            │
         ┌──────────────────────────────────────────────┐
         │ obtain greatest of maximum values for          ├── S428
         │ respective planes                              │
         └──────────────────┬───────────────────────────┘
                            │
         ┌──────────────────────────────────────────────┐
         │ calculate correction coefficients for          ├── S430
         │ respective planes                              │
         └──────────────────┬───────────────────────────┘
                            │
         ┌──────────────────────────────────────────────┐
         │ calculate radii from areas for images of the   ├── S432
         │ respective planes                              │
         └──────────────────┬───────────────────────────┘
                            │
         ┌──────────────────────────────────────────────┐
         │ calculate corrected radii for images of        │
         │ respective planes using correction             ├── S434
         │ coefficients and radii for the respective      │
         │ planes                                         │
         └──────────────────┬───────────────────────────┘
                            │
         ┌──────────────────────────────────────────────┐
         │ calculate average radius of corrected radii    │
         │ for images of respective planes                ├── S436
         └──────────────────┬───────────────────────────┘
                            │
         ┌──────────────────────────────────────────────┐
         │ calculate amount of urine in urinary bladder   ├── S438
         │ using average radius                           │
         └──────────────────┬───────────────────────────┘
                            │
                       ┌────┴────┐
                       │   END   │
                       └─────────┘
```

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20137995
- US 4926871 A
- US 2004267123 A1